# EUROPEAN PATENT SPECIFICATION

(11) **EP 3 319 513 B1**
(45) Date of publication and mention of the grant of the patent: **07.12.2022**
(21) Application number: 16822004.4
(22) Date of filing: 07.07.2016
(51) Int. Cl.: A61B 5/021, A61B 5/0215, A61B 5/0285, A61B 5/029, A61M 1/34, A61M 1/36, A61M 1/38, A61B 5/024, A61B 5/318, A61M 60/562, A61M 60/113

(54) **AUTOIMMUNE MECHANICAL IMMUNOMODULATION**
MECHANISCHE AUTOIMMUN-IMMUNMODULATION
IMMUNOMODULATION MÉCANIQUE AUTO-IMMUNE

(30) Priority: 07.07.2015 US 201562189678 P; 11.11.2015 US 201562254005 P; 05.02.2016 US 201662291973 P; 10.05.2016 US 201662334287 P
(43) Date of publication of application: 16.05.2018
(73) Proprietor: Khalil, Nashwa, Houston, TX 77075 (US)
(72) Inventor: Khalil, Nashwa, Houston, TX 77075 (US)
(74) Representative: Straus, Alexander
(86) International application number: PCT/US2016/041396
(87) International publication number: WO 2017/007969

(56) References cited:
- WO-A1-2014/147028
- WO-A1-2014/147028
- WO-A1-2015/099826
- US-A- 4 276 140
- US-A- 5 044 901
- US-A1- 2006 206 048
- US-A1- 2008 040 153
- US-A1- 2009 088 612
- US-A1- 2010 160 137
- US-A1- 2010 160 137
- US-A1- 2013 178 834
- US-A1- 2014 249 431
- US-B2- 8 529 489
- RYAN, J: 'Extracorporeal membrane oxygenation for pediatric cardiac arrest.' CRITICAL CARE NURSE vol. 35, no. 1, February 2015, pages 60 - 69, XP055347020

## Description

### BACKGROUND

Apheresis is a process in which the blood of a donor or patient is passed through an apparatus that separates out one or more components and returns the remainder to the patient. It is, therefore, an extracorporeal therapy. Therapeutic apheresis can be performed utilizing peripheral venous system to exchange three liters of plasma with three liters of an albumin solution. Patients are typically given 5- 15 therapeutic apheresis sessions based on a rough estimate from historical patient data for repressing symptomatic disease. Patients are treated for years this way because actual immune disease process has not been effectively treated.

Traditional methods of plasma exchange are performed using peripheral vasculature of the venous pathway. Only immune factors floating in the bloodstream are removed while immune factors concentrations and migrations within secondary lymphoid system are not changed. Therefore, the signaling pathways of the patient's immunological disease process do not change and patient's underlying immunological disease process remains constant.

There have been many attempts to improve efficacy of therapeutic apheresis, but, to date, none effectively change the migration of immune factors. Migration of immune factors relative to other immune factors in the tissue, vascular, and lymphatic system of the body effects functions of the overall immunological system. Conventional therapeutic apheresis offers, at best, only symptomatic relief without treatment of disease process. U.S. Patent Application Publication No. 2010/160137 A1 discloses a centrifuge for separating blood and blood components having a blood processing vessel mounted on a rotor of a centrifuge. A sensor in the outflow race of a return peristaltic pump detects air bubbles in the fluid within a return loop. U.S. Patent Application Publication No. 2008040153 A1 describes therapeutic apheresis treatments and diagnostic monitoring methods, specifically relating to the depletion and/or removal of a broad bandwidth of certain rheologically active elements from the blood of a patient followed by return of the blood so treated to the patient.

### SUMMARY

The present disclosure relates generally to a system for performing therapeutic apheresis. The present disclosure provides a system usable for a highly efficient form of therapeutic apheresis that modulates the immune system, thereby resulting in treatment of one or more immunological disease processes. The system is defined by the claims as below. The system comprises a pump, an apheresis centrifuge, a pulse detector, and a controller. The system is configured to receive arterial blood. Furthermore, the system is configured to return a portion of the blood in pulsatile flow via an arterial system of a patient.

The system, according to one embodiment of the present disclosure is capable of providing for returning to a patient in pulsatile flow at least a portion of the blood from an extracorporeal circuit. In other embodiments, the systems of the present disclosure utilize the central arterial system to exchange volumes of plasma to immunomodulate disease processes. The present disclosure is based, in part, on combining concepts of intermittent flow and continuous flow therapeutic apheresis along with utilizing established cardiovascular concepts. One benefit of the system suitable for therapeutic apheresis of the present disclosure is to decrease the amount of time spent by patients in therapeutic apheresis sessions, not only during initial treatment processes, but throughout the course of the patient's life. The term "patient" is broadly defined herein to include both humans and animals.

All disease states that have immune processes currently using traditional therapeutic apheresis methods can benefit from the therapeutic apheresis system described herein. In addition, other immunological disease processes that improve with immunomodulation, such as multi-organ transplant (including immunosuppression/rejection and induction/desenti-zation), established infections, allergies, needs of vaccine work such as inverse vaccine, and cancer states, would benefit from the therapeutic apheresis system described herein.

Due to improvement in efficacy of this form of apheresis, it may be used in pathology states in which avoiding immune complex injury (e.g., inflammation) and/or preventing the build-up of immune factors/inflammation is beneficial. The therapeutic apheresis of the present disclosure may prevent the resultant damage caused by such states; for example, in cardiac conditions in which immune pathology occurs.

The features and advantages of the present disclosure will be readily apparent to those skilled in the art upon a reading of the description of the embodiments that follows.

### DRAWINGS

The present disclosure contains at least one drawing executed in color. Copies of this patent or patent application publication with color drawing(s) will be provided by the Office upon request and payment of the necessary fee.

Some specific example embodiments of the disclosure may be understood by referring, in part, to the following description and the accompanying drawings.
Figure 1 is a schematic diagram of one possible example for using the system of the present disclosure.
Figure 2 is a schematic diagram showing additional details of the black box of the schematic diagram of Figure 1.

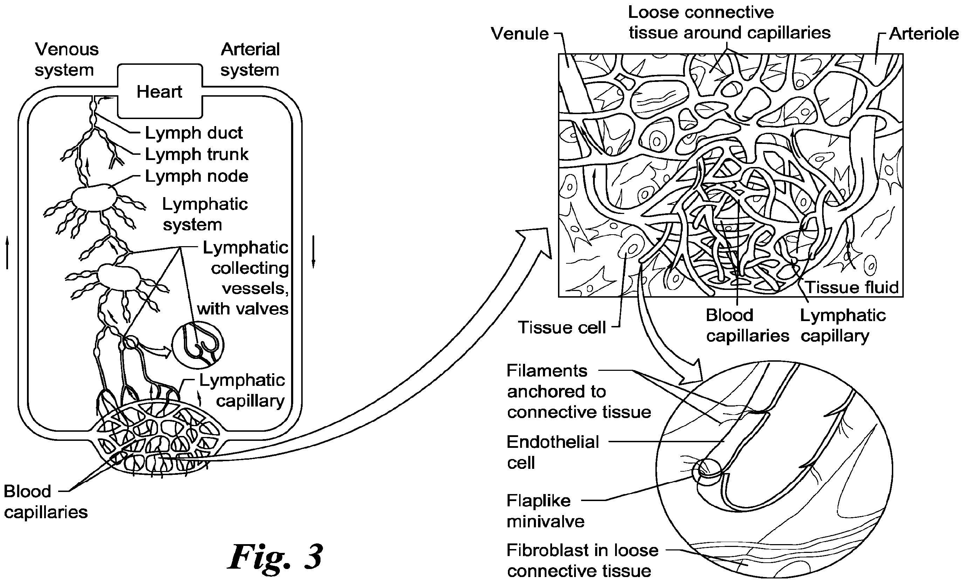

Figure 3 is a schematic diagram showing portions of the human venous and arterial systems.

While the present disclosure is susceptible to various modifications and alternative forms, specific example embodiments have been shown in the figures and are herein described in more detail. It should be understood, however, that the description of specific example embodiments is not intended to limit the invention to the particular forms disclosed.

### DESCRIPTION

The present disclosure relates generally to a system for performing therapeutic apheresis. The systems of the present disclosure provide a highly efficient form suitable for therapeutic apheresis to modulate the immune system, thereby resulting in treatment of one or more immunological disease processes. The therapeutic apheresis, of the present disclosure, is based, at least in part, on returning blood to a patient in pulsatile flow. As used herein, the term "blood" refers to whole blood or any substituent component of whole blood.

The systems of the present disclosure increase the velocity and pulsatility of arterial perfusion. Simultaneously, immune factors are removed from plasma of arterial blood, and blood that has been immune factor depleted is returned to the patient upstream in the central arterial system. This blood is returned at increased velocity and pulse to the capillary level/lymphatic system. The mechanism for transvasular flux of proteins, a shift of plasma proteins, is due to a decrease in plasma protein concentration that causes increase in lymph flow, which is greater than transvascular protein permeability. As a result, there is greater migration of immune factors from extravascular space into the intravascular space; and, once in the intravascular space, they can be removed via the continuous plasma exchange process of the present disclosure.

Overall, the systems of the present disclosure allow the humoral regulation pathways to be "reset" by mechanically augmenting hemodynamics of arterial flow while simultaneously removing immune factors from the arterial flow. When the flow and concentration of immune factors in the secondary lymphoid system that is responsible for signaling pathways of patient's current disease process are removed; than humoral immunity is changed. The body uses native modalities of migration pathways to return to standard humoral regulation in the extravascular.

In general, the present disclosure is directed to systems using an extracorporeal circuit configured to intake arterial blood from a patient, performing apheresis on the blood to remove one or more components from the blood, and returning in pulsatile flow at least a portion of the blood from the extracorporeal circuit. As used herein, "pulsatile flow" refers to flow with periodic variations in velocity (e.g., varying speeds of operation of a pump). Such systems may be used, among other things, to create an immunomodulatory effect on a patient's immune system. The term "immunomodulation" (and its derivatives) is broadly defined herein to encompass any adjustment of the immune response to a desired level, as well as all therapeutic interventions aimed at modifying the immune system (e.g., by inducing, enhancing, suppressing, and/or augmenting a patient's immune system). The therapeutic apheresis of the present disclosure may be combined with disease modifying treatment, such as immunosuppressive drugs or other therapeutic agents, suitable for use or treatment of the patient.

The extracorporeal circuit is configured to be operably connected to the circulatory system of a patient. For central arterial access to the aorta, a catheter can be inserted into the patient via the subclavian artery. Alternatively, a catheter can be inserted into the femoral artery, which is a pathway to the aorta. Arterial access for catheters can be radiologically guided and can follow standard arterial catheter access for vascular procedures. Similarly, a catheter can be inserted into one or more major anatomical branches of the central arterial system instead of the aorta, depending on the pathological state needing treatment. Different return blood flow velocities can be created or produced in the different major anatomical branches throughout the central arterial system, depending on the pathological states, such as neurological and/or orthopedic conditions, benefitting from this form of apheresis.

The extracorporeal circuit of the present disclosure includes an automated blood cell separator. The automated blood cell separator may be used to ensure fluid balance and maintain a normal plasma volume. Continuous flow centrifugation or intermittent flow centrifugation methods can be employed for apheresis. In some embodiments, the extracorporeal circuit includes an apheresis centrifuge.

The pulsatile flow from the extracorporeal circuit is provided by the force from one or more pumps.

The extracorporeal circuit of the present disclosure includes one or more pumps. In general, the pump is capable of returning to a patient at least a portion of the blood from an extracorporeal circuit. The pump also is capable of returning the portion of the blood in pulsatile flow. Suitable pumps include positive displacement pumps, roller pumps, ventricular pumps, piston pumps, kinetic pumps, centrifugal pumps, forced vortex pumps, magnetic pumps. In one embodiment, the pump may be disposed within an indwelling catheter. In certain embodiments, the pump may have an output that approximates systolic flow output of the left ventricle. In certain embodiments, the pump may have a variable displacement, timing, and fill/eject ratio control. The pumps can be configured to operate within a variable range, such that blood is pumped at a rate from approximately 0.1 liters/minute to as high as 1.5 liters/minute, 0.5 liters/minute to as high as 1 liter/minute, and 0.3 liters/minute to as high as 0.6 liters/minute. In one embodiment, one or more of the pumps can be extracorporeal or located at or near an end of an indwelling catheter, which is placed in the aorta or central arterial branches of the patient and is configured to deliver axial flow.

The pump is operatively connected to a controller. The controller is configured to produce one or more signals to the pump(s), and each pump is configured to act in accordance with information contained in each signal it receives from the controller. The controller controls the pump and allows for pulsatile flow of the portion of the blood returned to the patient form the extracorporeal circuit. In certain embodiments, the controller modulates the volumetric flow rate of the pump to coincide with the onset of ventricular systole. The controller also can receive inputs about native electrocardiogram of the patient or the paced rhythm. The controller can then provide information to the one or more pumps to effectuate return of blood to the patient via the arterial system. The control can also be configured to sense various pressures of patient (e.g., blood pressure) and decrease or increase the pump speed to compensate as necessary. The control can also be configured to sense other physiologic parameters, such as bioimpedence, body motion and/or cardiac performance parameters, to adjust pump speed to optimize flow of blood to the patient. The controller also may be operatively connected to other components of the systems of the present disclosure. For example, the controller may be configured to produce and/or receive signals from an automated blood cell separator, temperature sensors, pressure sensors, an air embolus sensor, pulse sensor (e.g., ECG) and/or a heat exchanger. Standard power delivery devices can be used in conjunction with the controller delivering proper wattage to allow operation of system.

In one embodiment, one or more of the signals from the controller can cause one or more of the pumps to pulsate blood flow to the patient. More particularly, one or more of the pumps can have the capacity to pump pulsatile blood flow to the patient, such that the pulsatile blood flow returns to the patient in axial flow. The controller can vary pump speed either in synchronization with the heart rhythm or a paced rhythm, or out of synchronization with the heart rhythm. In other words, the pulsation can mimic the rhythm of the patient's heart. Attempting to replicate the flow of blood to the patient with the patient's heart rate or a target heart rate is believed to improve the effectiveness of the treatment. By way of explanation, and not limitation, mathematical and physical models have shown that, at the same mean pressure, pulsatile blood flow has approximately 2.4 times the energy content of non-pulsatile flow, thereby dissipating significantly more energy through the tissue. Pulsation directly impacts blood flow within the lymphatic system, and energy from pulsatile flow on capillary and tissue exchanges influences what occurs with substances from tissues into the lymphatic system. In certain embodiments, the controller is configured such that the initiation of the ejection from the pump may occur less than or about 75 milliseconds after onset of systole as measured by ECG trace. The controller also may include a programmed delay to allow for tuning of the ejection start to the ECG signal being delivered.

In one embodiment, the control is configured to receive information about temperature of the blood and control a heat exchanger. Such a heat exchanger may be used to adjust the temperature of the blood returned to the patient. The controller, in conjunction with a heat exchanger, varies returned blood temperature either in synchronization with the current body, normal range temperature, or increased temperature. The heat exchanger can be used to ensure that the temperature of blood being returned to the patient is at an appropriate or acceptable temperature to reduce afterload resistance.

In one embodiment, velocity amplitudes of the blood flow returned to the patient can be modifying (e.g., increasing and/or decreasing) from baseline, while maintaining, increasing, or decreasing the baseline pulse frequency. In this way, different velocities in blood flow return may be created in order to effect changes in migration of components that affect immunomodulation.

The systems of the present disclosure also may include one or more pressure sensors, heat sensors, pulse sensors (e.g., electrocardiography (EKG) machine), or any combination thereof One or more heat exchangers can be used. Suitable heat exchangers may be able to heat at least a portion of the blood up to 5 degrees Fahrenheit above the normal body temperature range. In certain embodiments, a reservoir system may be used to facilitate processing of the blood and resultant components with respect to plasma exchange. In addition, a controller and/or monitor board can receive one or more patient inputs, such as blood properties, and track and/or display blood properties of the portion of the blood returned to the patient.

In one embodiment, the controllers configured, through an algorithm, to calculate the desired properties of the blood being returned to the patient. The algorithm's purpose is to evaluate the augmentation, changes, or modifications of blood properties desired. The algorithm can be subject to modification based on patient needs or plasma exchange properties based on, for example, the patient's physiological profile and/or disease. Plasma exchange properties relate to blood properties with regards to pulsatility, velocity of flow, and/or temperature.

Tubing or catheters used in the system of the present disclosure to move blood or other fluids can be made of any suitable bio-compatible or biomaterial. For example, plastic materials, such as those having the requisite strength and bio-compatibility characteristics and permits the function of directional flow, can be used. Rigid biomaterials or flexible biomaterials can alternatively or additionally be utilized for the construction. Examples of suitable tubing materials include those commonly used for extracorporeal circulation, such as apheresis, hemodialysis, cardiopulmonary bypass, and extracorporeal membrane oxygenation (ECMO).

In one embodiment, the systems of the present disclosure uses tubing materials having low dielectric constant, low coefficient of friction, non-reactivity, or combinations thereof Examples suitable tubing materials include polytetrafluoroethylene (PTFE) and perfluoroalkoxy (PF A).

As noted above, the systems of the present disclosure include an extracorporeal circuit for apheresis. In certain embodiments, such apheresis includes adding a replacement fluid to the blood. The replacement fluid can be fresh frozen plasma (FFP) and/or a predetermined percentage of albumin and/or saline solution. Heparin and/or saline can be added to the replacement fluid depending upon patient needs. Saline and/or heparin can be added to the procedure before and/or during the procedure. However, saline and heparin are not required to be added to the system, or can be added via peripheral venous vascular system. In one embodiment, saline can be added in amounts that are consistent with the industry standard of care and used for hydration, as understood by those skilled in the art. In one embodiment, patients are given heparin following standard anticoagulation given during vascular and plasma exchange procedures. Although hypocalcaemia due to citrate-based anticoagulants is a common minor adverse event of therapeutic apheresis, sodium citrate is widely accepted because healthy donors are able to handle it better. Heparin is better for sicker populations. Using heparin will keep static state of calcium ion activity. Citrate based anticoagulants bind with calcium ions. Decrease in calcium ions can cause increase in cardiac adverse events such as arrhythmias. For this reason, heparin is safely used in cardiac applications for many years. Heparin is standard of care for patients undergoing vascular procedures.

In one embodiment, the replacement fluid in the blood returning to the patient may include one or more therapeutic agents such as drug, immune factor, cell, nanoparticle, and/or vector. Replacement fluids that include therapeutic agents allow for enhanced delivery of such therapeutic agents. Replacement fluids including therapeutic agents may be exchanged as a full volume exchange or an exchange in any amount less than full volume. The length of time, amount of volume exchanged, ratio of exchange and concentrations of factors in constituents/replacement fluid returned per treatment in this therapeutic apheresis system will be dependent on the severity and pathology of disease process and the particular therapeutic agent, among other things.

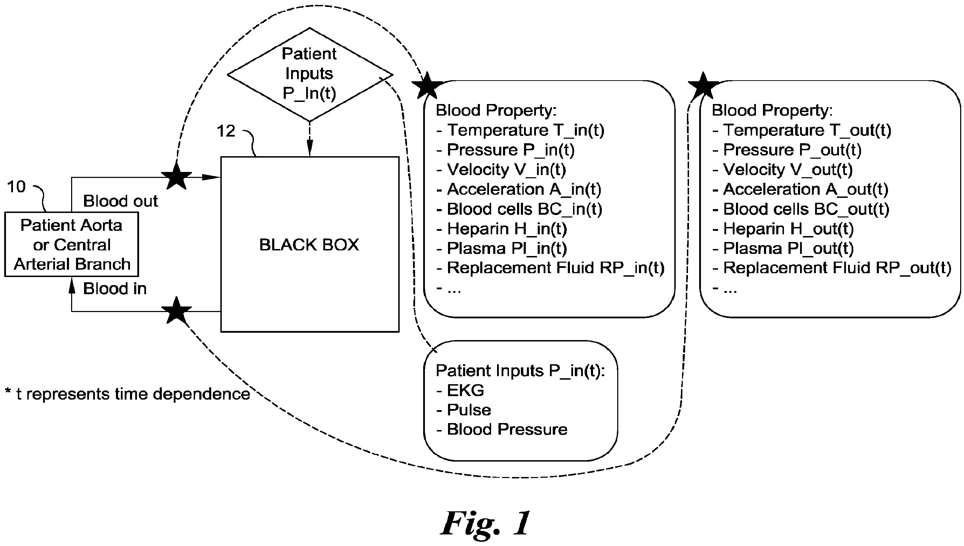

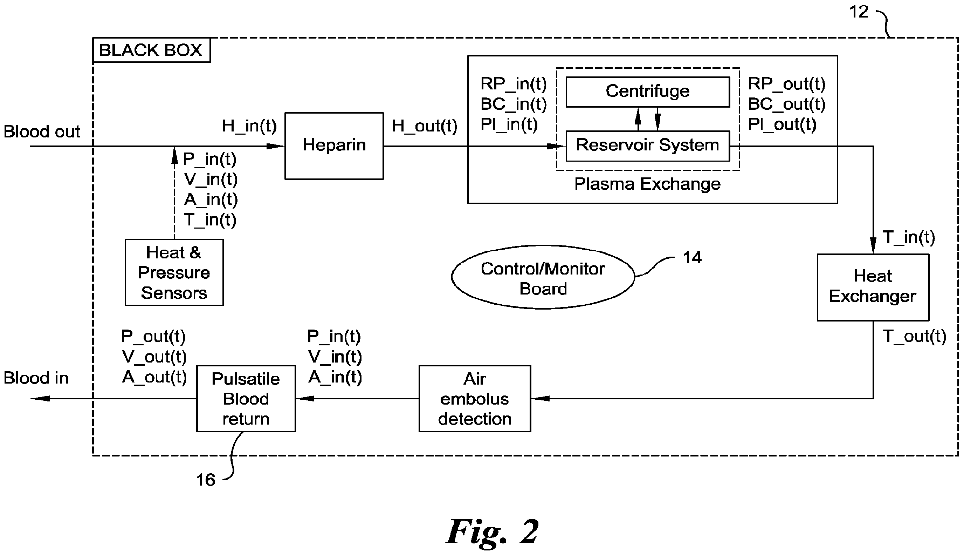

Figures 1 and 2 illustrate one possible example for using the system of the present disclosure. In particular, blood is received from the aorta or central arterial branches of the patient, generally designated 10, and transmitted to and/or through an extracorporeal circuit, generally designated 12. Certain properties of the blood, such as temperature (T), pressure (P), velocity (V), acceleration (A), number and/or type of certain blood cells (BC), heparin (H) levels, plasma (PI) levels and/or the like, is measured or analyzed during this transmission and/or prior to the blood being provided to the extracorporeal circuit 12. As described above, replacement fluid (RP) may be added to the blood prior to or after the blood being transmitted to the extracorporeal circuit 12. Certain information about the patient, such as blood pressure, pulse, heart rate and/or the like, is transmitted or provided to the extracorporeal circuit 12 or the controller 14 therein.

Blood that exits the extracorporeal circuit 12 is transmitted or provided back to the aorta or central arterial branches, for example, of the patient 10. Certain properties of the blood, such as temperature (T), pressure (P), velocity (V), acceleration (A), number and/or type of certain blood cells (BC), heparin levels (H), plasma levels (PI) and/or the like, are measured or analyzed during the transmission of the blood from the extracorporeal circuit 12 to the aorta or central arterial branches of the patient 10. The control or monitor board 14 employs the algorithm described above to control operation of the one or more pumps 16, to thereby pulsate the blood as it exits the extracorporeal circuit 12 and is returned to the patient. As shown in Figure 2, various parameters or characteristics (e.g., temperature) of the blood is monitored or measured throughout its transmission through the extracorporeal circuit 12.

To administer the treatment, a catheter can be placed as high as possible in the thoracic spine of the patient to get the neurostimulation benefit of blood supply to the spinal nervous system. Such placement is also above the renal arteries, which results in the benefit of blood supply to the renal system. Amplitude of pulsating flow is directly associated with enhanced renal flow, which results in improved organ function that will enhance body function between treatments.

The length of time and amount of volume exchanged per treatment will be dependent on the severity and pathology of immunological process needed to affect immunological niches. This is so that the self-perpetuated cycles of these immunological processes in the niches are broken before being able to build up more immunological factors. While a longer period of time per treatment may help allow the body time to catch up in fibrinogen, the more volume exchanged may require FFP. This procedure will be able to replace more than the standard 1-1.5 volume, as needed.

Notwithstanding that any numerical ranges and parameters setting forth the broad scope of the invention are approximations, the numerical values set forth in the specific examples are reported as precisely as possible. Any numerical value, however, inherently contain certain errors necessarily resulting from the standard deviation found in their respective testing measurements.

The system of the present disclosure can be stand-alone or operate separate and distinct from any other medical or healthcare system or apparatus. Alternatively, the system of the present disclosure can work in conjunction with one or more of hemodialysis, cardiopulmonary bypass, extracorporeal membrane oxygenation (ECMO), and/or aquapheresis.

Therefore, the present invention is well adapted to attain the ends and advantages mentioned as well as those that are inherent therein. Numerous changes may be made by those skilled in the art, which are encompassed within the appended claims.

## Claims

1. A system comprising: a pump; an apheresis centrifuge; a pulse detector; and a controller,
wherein the system is configured to receive arterial blood,
**characterized in that** the system is configured to return a portion of the blood in pulsatile flow via an arterial system of a patient.

2. The system of claim 1, further comprising a heat exchanger.

3. The system of claim 1, wherein the pulse detector is an electrocardiography (EKG) machine.

4. The system of claim 1, wherein the pump is one of a positive displacement pump or a kinetic pump.

5. The system of claim 1, wherein the pump is one or more of a positive displacement pump, a roller pumps, a ventricular pump, a piston pump, a kinetic pump, a centrifugal pump, a magnetic pump, and a forced vortex pump.

6. The system of claim 1,
further comprising electrical leads; or
further comprising one or more of a pressure sensor, a temperature sensor, and a pulse sensor.

## Patentansprüche

1. System, welches umfasst: eine Pumpe; eine Apherese-Zentrifuge; einen Pulsdetektor; und eine Steuerung,
worin das System ausgestaltet ist, arterielles Blut aufzunehmen,
**dadurch gekennzeichnet, dass** das System ausgestaltet ist, einen Teil des Blutes in pulsierendem Fluss über ein arterielles System eines Patienten zurückzuführen.

2. System nach Anspruch 1, welches ferner einen Wärmetauscher umfasst.

3. System nach Anspruch 1, worin der Pulsdetektor ein Elektrokardiographiegerät (EKG) ist.

4. System nach Anspruch 1, worin die Pumpe eine von einer Verdrängerpumpe oder einer kinetischen Pumpe ist.

5. System nach Anspruch 1, worin die Pumpe eine oder mehrere von einer Verdrängerpumpe, einer Rollenpumpe, einer Ventrikelpumpe, einer Kolbenpumpe, einer kinetischen Pumpe, einer Zentrifugalpumpe, einer magnetischen Pumpe und einer Festkörperwirbelpumpe ist.

6. System nach Anspruch 1,
welches ferner elektrische Leitungen umfasst; oder
welches ferner einen oder mehrere Drucksensoren, einen Temperatursensor und einen Pulssensor umfasst.

## Revendications

1. Système comprenant: une pompe; une centrifugeuse d'aphérèse; un détecteur de pouls; et une unité de contrôle,
dans lequel le système est configuré pour recevoir du sang artériel,
**caractérisé en ce que** le système est configuré pour renvoyer une partie du sang en flux pulsé via un système artériel d'un patient.

2. Système de la revendication 1, comprenant en outre un échangeur de chaleur.

3. Système de la revendication 1, dans lequel le détecteur de pouls est une machine d'électrocardiographie (EKG).

4. Système de la revendication 1, dans lequel la pompe est l'une parmi une pompe à déplacement positif ou une pompe cinétique.

5. Système de la revendication 1, dans lequel la pompe est une ou plusieurs d'une pompe à déplacement positif, d'une pompe à rouleaux, d'une pompe ventriculaire, d'une pompe à piston, d'une pompe cinétique, d'une pompe centrifuge, d'une pompe magnétique et d'une pompe à vortex forcé.

6. Système de la revendication 1,
comprenant en outre des fils électriques; ou
comprenant en outre un ou plusieurs des éléments suivants: un capteur de pression, un capteur de température et un capteur de pouls.
